(19) Europäisches Patentamt
European Patent Office
Office européen des brevets

(11) **EP 2 196 795 A1**

(12) **EUROPEAN PATENT APPLICATION**
published in accordance with Art. 153(4) EPC

(43) Date of publication:
**16.06.2010 Bulletin 2010/24**

(51) Int Cl.:
**G01N 21/64** (2006.01)

(21) Application number: **07806327.8**

(22) Date of filing: **30.08.2007**

(86) International application number:
**PCT/JP2007/066850**

(87) International publication number:
**WO 2009/028063 (05.03.2009 Gazette 2009/10)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Mitsui Engineering & Shipbuilding Co., Ltd.**
**Chuo-ku**
**Tokyo 104-8439 (JP)**

(72) Inventors:
• **KIMURA, Noriaki**
**Tamano-shi**
**Okayama 706-8651 (JP)**

• **NAKADA, Shigeyuki**
**Tamano-shi**
**Okayama 706-8651 (JP)**
• **DOI, Kyouji**
**Tamano-shi**
**Okayama 706-8651 (JP)**

(74) Representative: **Kastel, Stefan et al**
**Flügel Preissner Kastel Schober**
**Nymphenburger Strasse 20a**
**80335 München (DE)**

(54) **FRET DETECTION METHOD AND DEVICE**

(57)      When FRET (Fluorescence Resonance Energy Transfer) detection of a large number of samples is performed in a short time for a sample consisting of a donor molecule and an acceptor molecule, the donor molecule is irradiated at first with first laser light used for exciting a donor molecule subjected to intensity modulation at a frequency of f+Δf, the accepter molecule is irradiated with second laser light used for exciting an acceptor molecule subjected to intensity modulation at a frequency of f, and fluorescence emitted from the accepter molecule is received.

From a fluorescence signal thus received, a first signal component of fluorescence emitted from the accepter molecule through FRET, and a second signal component of fluorescence emitted from an accepter molecule excited through irradiation with the second laser light are extracted.

Phase lags of the first and second signal components thus extracted are then calculated and the presence of generation of FRET is judged based on these phase lags.

FIG.4

**Description**

FIELD OF INVENTION

The present invention relates to a method of and a device for detecting FRET (fluorescence resonance energy transfer) that energy of a first molecule transfers to energy of a second molecule, by receiving fluorescence emitted from the second molecule when the second molecule is excited by fluorescence of the first molecule to be excited by laser irradiation. Specifically, the present invention relates to a FRET detection technology for detecting interaction between a pair of a donor molecule (fluorescence molecule) and an acceptor molecule (fluorescence molecule) using fluorescence.

BACKGROUND ART

An analysis of protein function has been recently important as the post-genome related technology in the medical industry, the pharmaceutical industry and the food industry. Especially to analyze actions of cells, research is required for interactions (binding and separation) between a type of protein and another type of protein (or a low molecule compound), which are living substances in a living cell.
The interactions between a type of protein and another type of protein (or a low molecule compound) have been recently analyzed using a fluorescence resonance energy transfer (FRET) phenomenon. Interactions between molecules are herein detected within a range of several nanometers using fluorescence. The interaction detection using the FRET phenomenon is mainly performed with a microscope system.
Raid-open Japan Patent Publication No. JP-A-2005-207823 discloses a single molecule fluorescence analysis using the FRET phenomenon. According to the publication, a fluorescence correlation analysis method or a fluorescence intensity distribution analysis is performed based on received fluorescence using the FRET phenomenon. In the method, however, the number of samples (e.g., cells) is limited to at most several dozen for detecting FRET in a short period of time. In other words, it is difficult to statistically analyze a large number of cells as analysis targets in a short period of time.
Patent Document 1: Raid-open Japan Patent Publication No. JP-A-2005-207823

DISCLOSURE OF THE INVENTION

PROBLEM TO SOLVE

In view of the above, it is an object of the present invention to provide a method of and a device for detecting FRET in which a large number of samples, including donor molecules and acceptor molecules, can be analyzed in a short period of time.

MEANS FOR SOLUTION

To achieve the aforementioned object, the present invention provides a FRET detection method of detecting FRET (fluorescence resonance energy transfer) where energy of a first molecule transfers to a second molecule, by receiving a fluorescence emitted from the second molecule, the second molecule emitting the fluorescence when the second molecule is excited by a fluorescence emitted from the first molecule, the first molecule emitting the fluorescence when the first molecule is excited by irradiation of laser, the FRET detection method having the steps of:

   irradiating a first laser for exciting the first molecule and a second laser for exciting the second molecule, the first laser having an intensity modulated at a first frequency, the second laser having an intensity modulated at a second frequency, the second frequency being different from the first frequency;
   receiving the fluorescence emitted from the second molecule; and
   extracting first and second phase delays and determining whether or not an energy transfer from the first molecule to the second molecule is generated based on the first and second phase delays, in which the first phase delay represents a delay of a signal component at the first frequency, with respect to modulation of the intensity of the first laser, of a fluorescence signal obtained from the received fluorescence emitted from the second molecule, and the second phase delay represents a delay of a signal component at the second frequency, with respect to modulation of the intensity of the second laser, of the fluorescence signal obtained from the received fluorescence emitted from the second molecule.

The energy transfer is preferably determined based on a ratio of the first phase delay to the second phase delay. It is preferable that the intensity of the first laser is modulated using a first modulation signal having the first frequency for modulating the intensity of the first laser,

the intensity of the second laser is modulated using a second modulation signal having the second frequency for modulating the intensity of the second laser,
the first modulation signal having the first frequency is obtained by mixing the second modulation signal having the second frequency with a generation signal having a delta frequency, and
the fluorescence signal of the received fluorescence emitted from the second molecule is sampled using a frequency of integer multiple of the delta frequency as a sampling frequency in synchronization with the generation signal having the delta frequency. Then, a signal component of the first frequency in the fluorescence signal and a signal component of the second frequency in the fluorescence signal are preferably extracted by executing a frequency analysis for the sampled fluorescence signal using one integer-th of the delta frequency as a frequency resolution.

When the fluorescence emitted from the second molecule is received, the fluorescence emitted by the first molecule may be further received; a third phase delay of a fluorescence signal obtained from the received fluorescence emitted from the first molecule with respect to modulation of the intensity of the first laser, may be computed; and the third phase delay may be used for determining whether or not the FRET is generated.

The present invention provides a FRET detection device for detecting FRET where energy of a first molecule transfers to a second molecule, by receiving a fluorescence emitted from the second molecule, the second molecule emitting the fluorescence when the second molecule is excited by a fluorescence emitted from the first molecule, the first molecule emitting the fluorescence when the first molecule is excited by irradiation of laser, the FRET detection device comprising:

a laser light source unit for irradiating a first laser to the first molecule for exciting the first molecule and irradiating a second laser to the second molecule for exciting the second molecule;
a light receiving unit for receiving the fluorescence from the second molecule;
a light source control unit for generating modulation signals, for modulating an intensity of the first laser irradiated by the laser light source unit at a first frequency and for modulating an intensity of the second laser irradiated by the laser light source at a second frequency, the second frequency being different from the first frequency; and
a processing unit for extracting a first phase delay and a second phase delay and determining whether or not an energy transfer from the first molecule to the second molecule is generated based on the first and second phase delays, in which the first phase delay represents a delay of a signal component at the first frequency, with respect to modulation of the intensity of the first laser, of a fluorescence signal obtained from the received fluorescence emitted from the second molecule, and the second phase delay represents a delay of a signal component at the second frequency, with respect to modulation of the intensity of the second laser, of a fluorescence signal obtained from the received fluorescence emitted from the second molecule.

Then the processing unit preferably determines whether or not the energy transfer is generated based on a ratio of the first phase delay to the second phase delay. Preferably, the light source control unit modulates the intensity of the first laser using a first modulation signal having a first frequency for modulating the intensity of the first laser,
the light source control unit modulates the intensity of the second laser using a second modulation signal having a second frequency for modulating the intensity of the second laser,
the first modulation signal having the first frequency is obtained by mixing the second modulation signal having the second frequency with a generation signal having a delta frequency, and
the processing unit samples the fluorescence signal of the received fluorescence emitted from the second molecule using integer multiple of the delta frequency as a sampling frequency in synchronization with the generation signal having the delta frequency. Then, the processing unit preferably extracts a signal component of the fluorescence signal having the first frequency and a signal component of the fluorescence signal having the second frequency by executing a frequency analysis for the sampled fluorescence signal using one integer-th of the delta frequency as a frequency resolution.

Preferably, the light receiving unit preferably receives the fluorescence emitted from the first molecule in addition to the fluorescence emitted from the second molecule, and the processing unit computes a third phase delay and uses the third phase delay for determining whether or not FRET is generated, the third phase delay of a fluorescence signal obtained from the fluorescence, which is emitted from the first molecule and received by the light receiving unit, with respect to modulation of the intensity of the first laser.

[Advantageous Effects of the Invention]

According to the present invention, two types of lasers are used for exciting the first molecule and the second molecule, and intensities of the lasers are modulated at predetermined frequencies. In addition, frequencies are respectively shifted in the intensity modulations for discriminating different types of fluorescences. After reception of fluorescences to be emitted in response to the lasers, the phase delays are further computed for the fluorescences emitted from the second molecule at two frequencies using the fact that frequencies in the intensity modulations are different from each other. FRET is then detected based on the phase delays. The above signal processing can be executed in a short period of time. Therefore, the sample can be efficiently measured in a short period of time, and a result of FRET detection can be statistically established.

Especially, a ratio of the aforementioned two phase delays largely varies depending on generation of FRET. Accordingly, FRET can be precisely detected by using the ratio even if the FRET efficiency is low. Furthermore, when the present invention is applied to the flow cytometer, FRET can be detected by processing a signal of fluorescence emitted from the sample passing through a measurement point at a constant speed. Therefore, the sample can be efficiently measured in a short period of time, and a result of FRET detection can be statistically established. Moreover, interactions between target molecules can be efficiently researched.

BRIEF EXPLANATION OF DRAWINGS

[FIG.1] FIG.1 is a schematic configuration diagram of a flow cytometer, which is an embodiment of a FRET detection device of the present invention.
[FIG.2] FIG.2 is a schematic configuration diagram of a laser light source unit in the flow cytometer illustrated in FIG.1.
[FIG.3] FIG.3 is a schematic configuration diagram of a light receiving unit in the flow cytometer illustrated in FIG.1.
[FIG.4] FIG.4 is a schematic configuration diagram of a control and processing section in the flow cytometer illustrated in FIG.1.
[FIG.5] FIG.5 is a block diagram of a SSB modulator in the control and processing section illustrated in FIG.4.
[FIG.6] FIG.6 is a flowchart for illustrating a series of flow to be executed in an example FRET detection method of the present invention.
[FIG.7] FIG.7 is a chart for showing an example of an energy absorption spectrum and a fluorescence spectrum of a donor molecule and those of an acceptor molecule.
[FIG.8] FIG.8 is a diagram for illustrating generation of FRET

EXPLANATION OF REFERENCE NUMERALS

| 10 | Flow Cytometer |
|---|---|
| 12 | Sample |
| 20 | Detection section |
| 22 | Laser light source unit |
| 22a | Donor excitation light source |
| 22b | Acceptor excitation light source |
| 23a, 23b | Lens systems |
| 24,26 | Light receiving units |
| 26a | Lens system |
| 26b | Dichroic mirror |
| $26c_1$, $26c_2$ | Band-pass filters |
| 27a, 27b | Photoelectric converters |
| 28 | Control and processing section |
| 30 | Tube line |
| 32 | Recovery container |
| 34a, 34b | Laser drivers |
| 40 | Signal generation unit |
| 42 | Signal processing unit |
| 44 | Controller |
| 46, 47 | Oscillators |
| 48, 56 | Power splitters |
| 50 | SSB modulator |
| 50a9 | 90 degree hybrid |
| 50b, 50c | Mixers |
| 50d | 90 degree phase shifter |
| 52, 54a, 54b, 64 | Amplifiers |

| 58a, 58b | IQ mixers |
| 60 | System controller |
| 62 | Low-pass filter |
| 66 | A/D converter |
| 80 | Analysis device |

BEST MODE FOR CARRYING OUT THE INVENTION

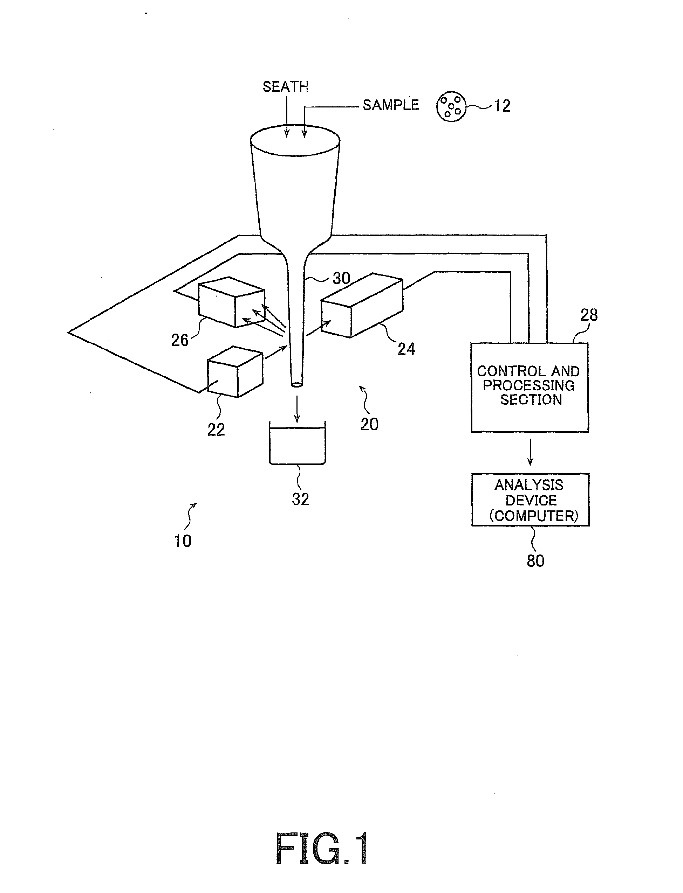

A method of and a device for detecting FRET of the present invention will be hereinafter explained in detail. FIG.1 is a schematic configuration diagram of a flow cytometer 10, which is an embodiment of a FRET detection device of the present invention.

The flow cytometer 10 is configured to determine whether or not energy of a first molecule (donor molecule) transfers to energy of a second molecule (acceptor molecule) by receiving fluorescence emitted from the acceptor molecule when the acceptor is excited by fluorescence emitted from the donor molecule. The donor molecule emits fluorescence when being excited by laser irradiation. To detect the energy transfer, intensity modulation is executed for lasers to be irradiated respectively to the donor molecule and the acceptor molecule at predetermined frequencies. The present invention has a feature that FRET generation is detected by measuring phase delays of fluorescences to be emitted in response to laser irradiation as described below.

The flow cytometer 20 includes a detection section 20 and an analysis device (computer) 80. The detection section 20 flows a sample 12 at a constant speed and irradiates laser to the sample 12. The sample 12 herein contains donor molecules and acceptor molecules, and the molecules emit fluorescence in response to laser irradiation. The detection section 20 detects a fluorescence signal of fluorescence emitted from the sample 12. The analysis device detects whether or not FRET is generated in the sample 12 based on a processing result obtained by the detection section 20.

The detection section 20 includes a laser light source unit 22, light receiving units 24, 26, a control and processing section 28 and a tube line 30. The control and processing section 28 includes a control unit and a processing unit. The control unit modulates intensity of laser to be irradiated from the laser light source unit 22 at a predetermined frequency. The processing unit processes a fluorescence signal from the sample 12 which flows through the tube line 30 with sheath liquid that forms a high speed flow, thereby forming a flow cell. In addition, a recovery container 32 is disposed at the outlet of the tube line 30.

The laser light source unit 22 is composed of a donor excitation light source 22a and an acceptor excitation light source 22b. The light source 22a emits laser with a predetermined wavelength. When absorbing the laser, the donor molecule enters an excited state. On the other hand, the light source 22b emits laser with a predetermined wavelength. When absorbing the laser, the acceptor molecule enters an excited state.

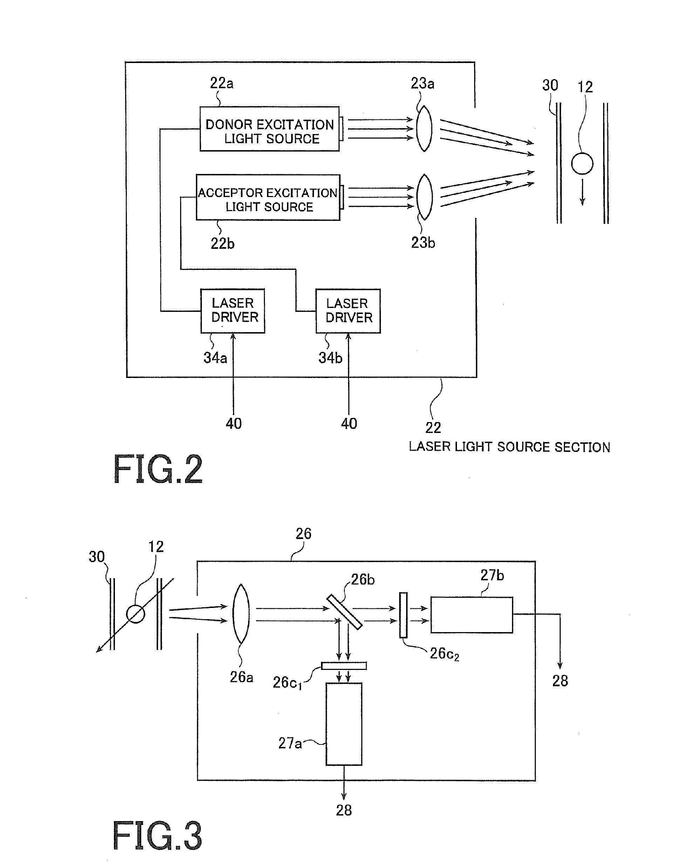

FIG.2 is a schematic configuration diagram of the laser light source unit 22.

The donor excitation light source 22a is a light source for emitting laser to excite the donor molecule. On the other hand, the acceptor excitation light source 22b is a light source for emitting laser to excite the acceptor molecule. Wavelength bands of the lasers for exciting the donor molecule and the acceptor molecule are different from each other. Accordingly, the laser for exciting the donor molecule can suitably excite the donor molecule whereas the laser for exciting the acceptor molecule can suitably excite the acceptor molecule. For example, when CFP (cyan fluorescent protein) is used for the donor molecule, laser with wavelength of 405-440nm is used. On the other hand, when YFP (yellow fluorescent protein) is used for the acceptor molecule, laser with wavelength of 470-530nm is used. The laser light source unit 22 is a unit for emitting each laser with a continuous wavelength of a visible-light band under the condition that its intensity is modulated at a predetermined frequency.

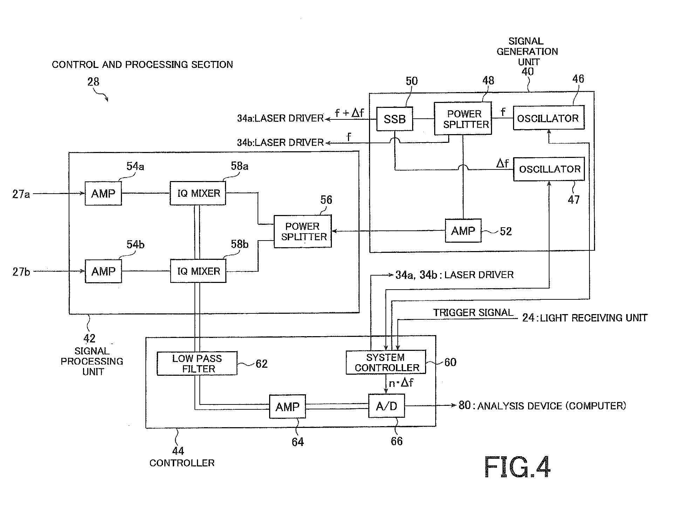

Optical lenses 23a, 23b are disposed on the light paths of the light sources, respectively. The optical lenses 23a, 23b focus lasers on the identical position. The sample 12, a measurement target, passes through the focal position. Laser drivers 34a, 34b are connected to the laser light sources, respectively. The laser drivers 34a, 34b drive the light sources, respectively, for causing them to emit laser. The laser drivers 34a, 34b are controlled to modulate intensities of lasers in response to signals from a signal generation unit 40 (see FIG.4) to be described.

For example, semiconductor laser units are employed as the donor and acceptor excitation light sources 22a, 22b for emitting lasers. For example, the semiconductor laser units emit lasers with the output power of about 5-100mW. For example, frequency (modulation frequency) is set to be 10-100 MHz in the modulation of laser intensity. A cycle of the intensity modulation is herein longer than a fluorescence relaxation time of fluorescence emitted from the donor molecule and that of fluorescence emitted from the acceptor molecules. In the intensity modulation, frequencies of the lasers for exciting the donor molecule and the acceptor molecule are different from each other by 100 kHz to 2 MHz. In the intensity modulation, the frequency of the laser for exciting the donor molecule is higher than that of the laser for exciting the acceptor molecule. In the present invention, however, the frequency of the laser for exciting the acceptor molecule may be higher than that of the laser for exciting the donor molecule in the intensity modulation. As described below, the reason why frequencies of lasers are slightly different in the intensity modulation is to process fluorescence signals of the received fluorescence in a short period of time.

The laser light source 22 oscillates at predetermined wavelength bands. Accordingly, the donor molecule and the acceptor molecule respectively emit fluorescences with predetermined wavelength bands when being excited by lasers, respectively. In the sample 12, the donor and acceptor molecules herein emit fluorescences in response to laser irradiation. When the sample 12 passes through the tube line 30, laser is irradiated to the sample 12 at the measurement point. The sample 12 accordingly emits fluorescence with a wavelength peculiar to the fluorescence molecule.

The light receiving unit 24 is opposed to the laser light source unit 22 through the tube line 30. The light receiving unit 24 is provided with a photoelectric converter.

In response to laser forwardly scattered by the sample 12 passing through the measurement point, the photoelectric converter outputs a detection signal for indicating that the sample 12 passes through the measurement point. The signal to be outputted by the light receiving unit 24 is provided to the control and processing section 28. In the control and processing section 28, the signal is used as a trigger signal for informing a timing that the labeled sample 12 passes through the measurement point in the tube line 30.

On the other hand, the light receiving unit 26 is disposed in a direction perpendicular to both of a laser beam direction of the laser irradiated by the laser light source unit 22 and a moving direction of the sample 12 in the tube line 30. A photoelectric converter is used as the light receiving unit 26. The photoelectric converter receives fluorescence emitted from the sample 12 irradiated at the measurement point. A photomultiplier and an avalanche photodiode are examples of the photoelectric converter.

FIG.3 is a schematic configuration diagram for illustrating an example schematic configuration of the light receiving unit 26.

In FIG.3, the light receiving unit 26 includes a lens system 26a, a dichroic mirror 26b, band-pass filters $26c_1$, $26c_2$, and photoelectric converters 27a, 27b (e.g., a photomultiplier(s) and/or an avalanche photodiode(s)). The lens system 26a focuses fluorescence signals of fluorescences from the sample 12.

After fluorescence entered the light receiving unit 26, the lens system 26a focuses the fluorescence on the light receiving surface of the photoelectric converters 27a, 27b.

The dichroic mirror 26b is a mirror which has wavelength characteristics set for reflection and transmission, respectively. Accordingly, fluorescence emitted from the acceptor molecule transmits through the mirror, but fluorescence emitted from the donor molecule is reflected by the mirror. The band-pass filter $26c_1$ filters fluorescence, whereby the photoelectric converter 27a then takes a fluorescence component of a predetermined wavelength band in the fluorescence emitted from the donor molecule. On the other hand, the band-pass filter $26c_2$ filters fluorescence, whereby the photoelectric converter 27b then takes a fluorescence component of a predetermined wavelength band in the fluorescence emitted from the acceptor molecule. Therefore, the photoelectric converter 27a receives fluorescence emitted from the donor molecule, whereas the photoelectric converter 27b receives fluorescence emitted from the acceptor molecule.

The band-pass filters $26c_1$, $26c_2$ are disposed in front of the light receiving planes of the photoelectric converters 27a, 27b, respectively. Each of the respective band-pass filters $26c_1$, $26c_2$ is a filter for only allowing transmission of specific fluorescence with a predetermined wavelength band. The wavelength bands of fluorescences, allowed to transmit through the filters, are correspondingly set to wavelength bands of fluorescences emitted from the donor molecule and the acceptor molecule. The wavelength bands are herein different from each other.

Each of the photoelectric converters 27a, 27b is a sensor for converting light received by its photoelectric surface into an electric signal. Each of the photoelectric converters 27a, 27b is provided with a sensor including e.g., a photomultiplier. In this case, fluorescences to be herein received are phase-delayed fluorescences to be emitted in response to irradiation of intensity-modulated laser. The fluorescences are received as light signals having phase-delay information. Therefore, electric signals to be outputted are fluorescence signals having signal information about phases. The fluorescence signal is provided to the control and processing section 28. Then, the fluorescence signal is amplified by an amplifier.

As illustrated in FIG.4, the control and processing section 28 is composed of a signal generation unit 40, a signal processing unit 42 and a controller 44. The signal generation unit 40 forms a light source control unit of the present invention for generating a modulation signal with a predetermined frequency. Additionally, the controller 44 and an analysis device 80 to be described form a processing unit of the present invention.

The signal generation unit 40 is a unit for generating a modulation signal to modulate (amplitude-modulate) intensity of laser at a predetermined frequency.

Specifically, the signal generation unit 40 includes oscillators 46, 47, a power splitter 48, a SSB (single side band) modulator 50 and an amplifier 52. The signal generation unit 40 provides generated modulation signals to the drivers 34a, 34b of the laser light source unit 22, as well as the signal processing unit 42. As described below, a modulation signal is provided to the signal processing unit 42, so that the modulation signal can be used as a reference signal for detecting phases of fluorescence signals to be outputted by the photoelectric converters 27a, 27b. The modulation signal is a sinusoidal wave signal with a predetermined frequency. In this case, the frequency is set to be in a range of 10-100 MHz. Describing a frequency of a modulation signal to be provided to the laser driver 34b as "f", the frequency f is a frequency of a modulation signal of the laser for exciting the acceptor molecule, which is set to be 10-100 MHz, as described above. On the other hand, a frequency of a modulation signal to be provided to the laser driver 34a is a frequency of the laser for exciting the donor molecule, which is defined as "f+$\Delta$f". In this case, $\Delta$f is set to be 100 kHz to 2 MHz, which is smaller than f = 10-100 MHz.

The oscillator 46 generates a sinusoidal wave signal with the frequency f, whereas the oscillator 47 generates a sinusoidal wave signal with the frequency $\Delta$f. The SSB modulator 50 generates a sinusoidal wave signal with the frequency f+$\Delta$f by integrating a sinusoidal wave signal with the frequency f and a sinusoidal wave signal with the frequency $\Delta$f. The sinusoidal wave signal with the frequency f+$\Delta$f is an integrated signal shifted towards the high-frequency direction (USB signal). The sinusoidal wave signal is provided to the laser driver 34a as a modulation signal. On the other hand, a sinusoidal wave signal with the frequency f is provided from the power splitter 48 to the laser driver 34b as a modulation signal.

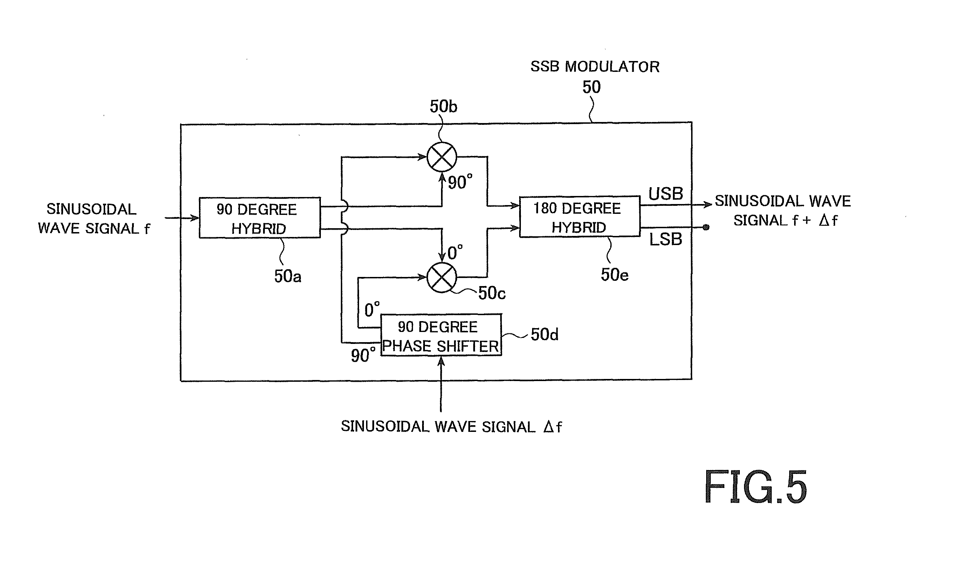

FIG.5 is a block diagram of the SSB modulator 50.
The SSB modulator 50 is composed of a 90 degree hybrid 50a, mixers 50b, 50c, a 90 degree phase shifter 50d, and a 180 degree hybrid 50e.
The 90 degree hybrid 50a is a high-frequency device provided with a hybrid ring. The hybrid ring divides a sinusoidal wave signal with the frequency f, which is provided from the oscillator 46, into 0-degree and 90-degree phased signals. The 90-degree hybrid 50a provides a divided 0-degree phased sinusoidal wave signal to the mixer 50c, whereas it provides a divided 90-degree phased sinusoidal wave signal to the mixer 50b. On the other hand, the 90 degree phase shifter 50d shifts a phase of a sinusoidal wave signal with the frequency $\Delta$f provided from the oscillator 47 by 0 degree and 90 degree. The 90 degree phase shifter 50d then provides the sinusoidal wave signals resulted to the mixers 50b, 50c, respectively.
The 180 degree hybrid 50e is a high-frequency device provided with a hybrid ring. The hybrid ring divides a received signal into 0-degree and 180-degree phased signals. In one terminal of the 180-degree hybrid 50e, a USB signal is generated by shifting a frequency of the sinusoidal wave signal by $\Delta$f towards a higher frequency direction while phase information of the sinusoidal wave signal is maintained. In the other terminal of the 180-degree hybrid 50e, a LSB signal is generated by shifting a frequency of the sinusoidal wave signal by $\Delta$f towards a lower frequency direction while phase information of the receiving signal is maintained.
The SSB modulator 50 outputs and provides the USB signal to the laser driver 34a.

The signal processing unit 42 is a unit for extracting information of a phase delay(phase difference) of fluorescence emitted from the sample 12 in response to laser irradiation, while using fluorescence signals to be outputted by the photoelectric converters 27a, 27b. The signal processing unit 42 is composed of amplifiers 54a, 54b, a power splitter 56 and IQ mixers 58a, 58b. The amplifiers 54a, 54b amplify the fluorescence signals to be outputted by the photoelectric converters 27a, 27b, respectively. The power splitter 56 splits the modulation signal, which is the sinusoidal wave signal of frequency f provided by the signal generation unit 40. The power splitter 56 provides the split signals to the respective amplified fluorescence signals. Each of the IQ mixers 58a, 58b mixes the amplified fluorescence signal with the split modulation signal.

The IQ mixer 58a/58b is provided correspondingly to the photoelectric converter 27a/27b for mixing the fluorescence signal of fluorescence emitted from the donor/acceptor molecule, which is provided by the photoelectric converter 27a/27b, with the split modulation signal to be provided by the signal generation unit 40 as a reference signal in a synchronization manner. As described below, a phase delay of fluorescence can be obtained by mixing the fluorescence signal with the modulation signal as a reference signal in a synchronization manner.
Specifically, each of the IQ mixers multiplies the reference signal and the fluorescence signal (RF signal) to compute a processing signal that includes a cos component (real part) and a high-frequency component of the fluorescence signal. Simultaneously, each of the IQ mixers multiplies the fluorescence signal and a signal obtained by shifting a phase of the reference signal by 90 degrees to compute a processing signal that includes a sin component (imaginary part) and a high-frequency component of the fluorescence signal. The aforementioned pair of processing signals is either of fluorescence signals of two types, that is, a signal of fluorescence emitted from the donor molecule and a signal of fluorescence emitted from the acceptor molecule. The processing signals having the cos component and that having the sin component are provided to the controller 44.

The controller 44 is a unit for controlling the signal generation unit 40 to generate a sinusoidal signal of frequency f and that of frequency $\Delta$f. The controller 44 is also a unit for obtaining the cos component and the sin component of the fluorescence signal by removing the high-frequency component from each of the processing signals having the cos component and the sin component of the fluorescence signal, which are obtained by the signal processing unit 42.

Specifically, the controller 44 is composed of a system controller 60, a low-pass filter 62, an amplifier 64 and an A/D converter 66. The system controller 60 gives an instruction for controlling actions of portion of the section. Additionally, the system controller 60 controls and manages all the actions of the flow cytometer 10. The low-pass filter 62 removes the high-frequency component from the respective processing signals in which the high-frequency component is added to the cos component and the sin component. Here, the processing signals are computed by the signal processing unit 42. The amplifier 64 amplifies the respective processing signals in which the cos component and the sin component are included but the high-frequency component is removed. The A/D converter 66 samples the respective amplified processing signals. The A/D converter 66 samples the respective processing signals having the cos component and the sin component, which the high-frequency element is removed therefrom. The A/D converter 66 then provides the sampled signal to the analysis device 80. In the sampling, integer multiple (preferably triple or more) of the aforementioned frequency $\Delta f$ is set to be a sampling frequency. The low-pass filter 62 has frequency characteristics set for anti-aliasing in digitalization by the A/D converter 66 and for allowing a signal component of frequency $\Delta f$ to pass through it.

The analysis device 80 executes a frequency analysis for the processing signals provided thereto, in which a frequency resolution is one integer-th of $\Delta f$, while the processing signal is synchronized with the aforementioned sinusoidal wave signal of frequency $\Delta f$. The analysis device 80 then computes an amplitude and a phase of the processing signals at frequency 0 Hz and those of the processing signals at frequency $\Delta f$. The computed phase is obtained based on the processing signals obtained by mixing the fluorescence signals with the sinusoidal wave signal of frequency f as a reference signal as described above. Therefore, the phases are phase delays with respect to the aforementioned sinusoidal wave signal (laser intensity modulation). As described above, the processing signals are composed of signals of two types. One is originated from the fluorescence signal of fluorescence emitted from the donor molecules. The other is originated from the fluorescence signal of fluorescence emitted from the acceptor molecules. Therefore, the amplitude and the phase of the processing signal at frequency 0 Hz and those of the processing signal at frequency $\Delta f$ are separately extracted for separating the fluorescence signals of two types.

The analysis device 80 computes phases of the processing signal of fluorescence emitted from the acceptor molecule at frequencies 0 Hz and $\Delta f$. Then, the analysis device 80 computes a ratio of the phase at frequency $\Delta f$ to that at frequency 0 Hz. The analysis device 80 detects FRET based on magnitude of the ratio. In other words, the analysis device 80 determines whether or not the aforementioned FRET is generated. For example, the analysis device 80 determines that FRET is generated when the aforementioned ratio exceeds a preliminarily set threshold.

The following is the reason to thus use the phases of the processing signal of fluorescence emitted from the acceptor molecule at frequencies 0 Hz and $\Delta f$. The phase of the processing signal at 0 Hz indicates a phase delay of fluorescence emitted from acceptor molecule in response to irradiation of laser whose intensity is modulated at frequency f. On the other hand, the phase of the processing signal at frequency $\Delta f$ indicates a phase delay due to generation of FRET, that is a phase delay of fluorescence emitted from the acceptor molecule excited when it absorbs fluorescence (as excitation light) emitted by the donor molecule in response to irradiation of laser whose intensity is modulated at frequency f+$\Delta f$.

As described below, the phase of the processing signal at frequency $\Delta f$, that is, the phase delay to be caused by generation of FRET, is expressed with fluorescence emission speed of fluorescence emitted from the donor molecule in response to laser irradiation, generation speed of FRET, fluorescence emission speed of fluorescence emitted from the acceptor molecule excited through FRET, and fluorescence emission speed of fluorescence emitted from the acceptor molecule in response to laser irradiation. On the other hand, as described below, the phase of the processing signal at frequency 0 Hz, that is, the phase delay of fluorescence to be emitted in response to laser irradiation is expressed with fluorescence emission speed of fluorescence emitted from the acceptor molecule in response to laser irradiation. Therefore, as described below, it is possible to determine whether or not FRET is generated by obtaining the phase delay ratio. Especially, a value of a FRET speed is often much smaller than that of the fluorescence emission speed of the donor molecule and that of the fluorescence emission speed of the acceptor molecule. Furthermore, when the emission speed of the donor molecule and that of the acceptor molecule are relatively identical to each other, a value of the aforementioned ratio will be 2. Therefore, the analysis device 80 can relatively easily detect FRET generation based on the aforementioned ratio by computing the phase delay ratio that is a measurement result.

When FRET is generated, a phase at frequency $\Delta f$ Hz of the processing signal of fluorescence emitted from the donor molecule varies in accordance with FRET generation. It is also possible to determine whether or not FRET is generated using the phase variation. In other words, a phase delay, corresponding to intensity modulation of laser for exciting the donor molecule, is extracted from the fluorescence signal of fluorescence emitted from the donor molecule. The phase delay can be then used for detecting FRET generation.

Obviously, the analysis device 80 can quantitatively calculate intensity of fluorescence emitted from the donor molecule and that of fluorescence emitted from the acceptor molecule using amplitudes of the processing signal at frequencies 0 Hz and $\Delta f$. Furthermore, FRET generation can be detected based on intensity of fluorescence at frequency $\Delta f$.
The above is the explanation of the flow cytometer 10 for embodying the present invention.

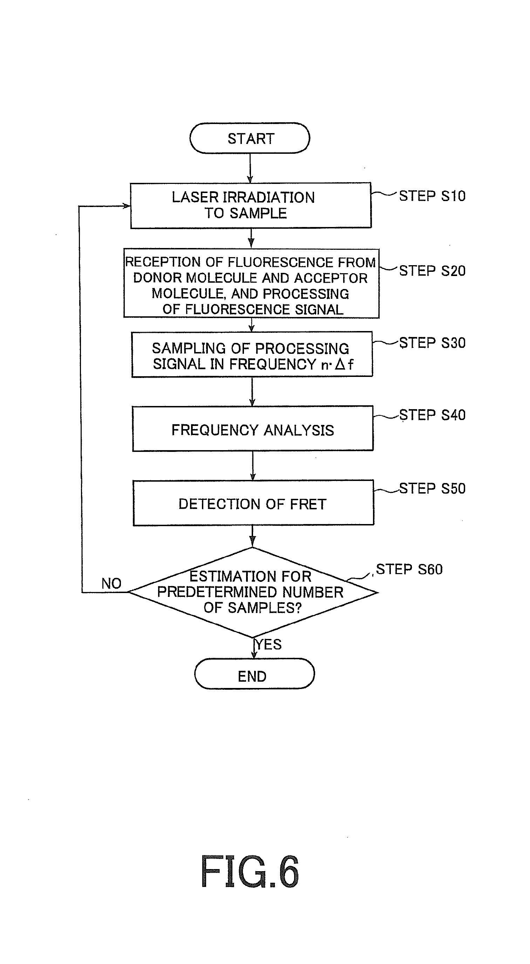

Next, a FRET detection method to be executed by the flow cytometer 10 will be hereinafter explained. FIG.6 is a flowchart for illustrating a series of flow for detecting FRET generation.

First, the controller 44 sends an instruction to the oscillator 46, and causes it to generate a sinusoidal wave signal of frequency f. The controller 44 also sends an instruction to the oscillator 47, and causes it to generate a sinusoidal wave signal of frequency $\Delta f$. The sinusoidal wave signal of frequency f is split in two signals by the power splitter 48.

One is provided to the SSB modulator 50, whereas the other is provided to the amplifier 52. The sinusoidal wave signal of frequency Δf is provided to the SSB modulator 50.

In the SSB modulator 50, the provided sinusoidal wave signal of frequency f is mixed with the sinusoidal wave signal of frequency Δf. Accordingly, a sinusoidal wave signal of frequency f+Δf, an mixed signal on the higher frequency side, is generated. The sinusoidal wave signal of frequency f+Δf is then provided to the laser driver 34a as an intensity modulation signal.

On the other hand, the sinusoidal wave signal of frequency f, the rest of the signals after splitting the sinusoidal wave signal by the power splitter 48, is also provided to the laser driver 34b as an intensity modulation signal.

Under the condition, the sample 12 flows through the tube line 30 and a sheath flow is formed. For example, the sheath flow flows through a flow path with the diameter of 100μm at the speed of 1-10m/second. The sample 12 contains the donor molecules and the acceptor molecules, both of which emit fluorescence in response to laser irradiation.

The donor excitation light source 22a irradiates laser whose intensity is modulated at frequency f+Δf. On the other hand, the acceptor excitation light source 22b irradiates laser whose intensity is modulated at frequency f. These lasers are simultaneously irradiated to the sample 12 (Step S10).

Laser irradiation is focused on a measurement point. When the light receiving unit 24 detects passage of the sample 12, a detection signal is outputted to the controller 44 as a trigger signal.

On the other hand, the photoelectric converter 27a starts receiving fluorescence emitted from the donor molecule whereas the photoelectric converter 27b starts receiving fluorescence emitted from the acceptor molecule. Using the above detection signal as a trigger signal, the signal processing unit 42 and the controller 44 executes signal processing for the fluorescence signals received by the photoelectric converters 27a, 27b (Step S20).

Specifically, each of the IQ mixers 54a,54b mixes the fluorescence signal with the sinusoidal wave signal of frequency f (as a reference signal) that is provided from the amplifier 52. Accordingly, each of the IQ mixers produces a processing signal that a high-frequency component is added to the cos component and the sin component. The processing signals are provided to the controller 44.

The controller 44 performs filtering processing. Specifically, the high-frequency components are removed from the respective processing signals that the high-frequency components are added to the cos components and the sin components. Accordingly, the processing signals, each of which is composed of the cos component and the sin component of the fluorescence signal, are extracted. The extracted signals are provided to the A/D converter 66 via the amplifier 64.

In this case, a signal of frequency n·Δf is provided to the system controller 60 of the controller 44. The frequency n·Δf is herein integer-multiple of frequency Δf of the sinusoidal wave signal to be generated by the oscillator 47. The A/D converter 66 samples the processing signal of fluorescence emitted from the donor molecule and that of fluorescence emitted from the acceptor molecule in synchronization with the signal of frequency n·Δf (Step S30).

Thus digitalized processing signal is provided to the analysis device 80.

The analysis device 80 executes a frequency analysis with respect to the sampled processing signal of fluorescence emitted from the acceptor molecule (Step S40). The frequency analysis is executed in a frequency resolution of one integer-th of Δf. After execution of the frequency analysis, a component of the processing signal at frequency 0 Hz is a fluorescence signal component of fluorescence emitted from the acceptor molecule in response to irradiation of laser. On other hand, a component of the processing signal at frequency Δf is a fluorescence signal component of fluorescence emitted from the acceptor molecule through FRET. The fluorescence signal components are thus extracted by using different frequencies at which intensity of laser is modulated.

Based on the result obtained by the frequency analysis, computation is executed for a ratio of a phase delay at frequency Δf Hz of the processing signal of fluorescence emitted from the acceptor molecule to a phase dlay at frequency 0 Hz of the processing signal of fluorescence emitted from the acceptor molecule. FRET detection is executed by comparing the ratio with a preliminarily set threshold (Step S50). Specifically, FRET generation is determined when the computed ratio exceeds the threshold.

Alternatively, FRET detection can be comprehensively performed by computing and using a phase delay ratio of the processing signal of fluorescence emitted from the donor molecule in addition to the processing signal of fluorescence emitted from the acceptor molecule.

Finally, it is determined whether or not the number of samples, which has passed through a measurement point in the flow cytometer 10, reaches a predetermined number (e.g., 1,000). Measurement will be continued until the number of samples reaches the predetermined number.

In a short period of time, it is possible to determine whether or not FRET is generated and to efficiently detect FRET in the sample passing through the measurement point by:

irradiating laser whose intensity is modulated to the donor molecule in the sample; receiving fluorescence emitted from the acceptor molecule; executing signal processing; and computing a phase delay corresponding to laser intensity modulation.

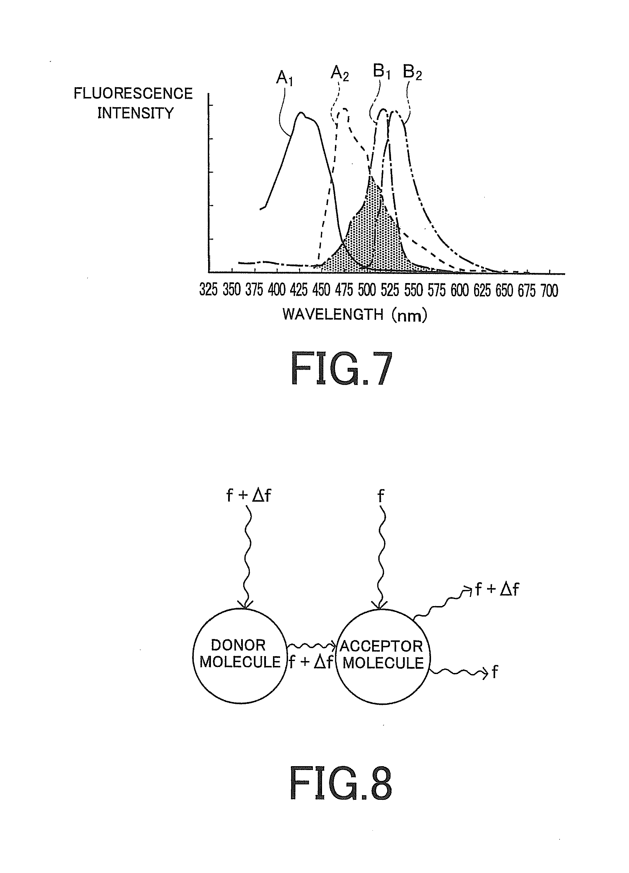

FIG.7 is a chart for showing an example of an energy absorption spectrum and a fluorescence spectrum of the donor molecule and those of the acceptor molecule. FIG.8 is a diagram for illustrating FRET generation in an easy understanding manner.

FIG.7 shows characteristics of energy absorption and fluorescence emission of the donor molecule and the acceptor molecule. In this case, the donor molecule is CFP (cyan fluorescent protein) whereas the acceptor molecule is YFP (yellow fluorescent protein). In FIG.7, curves are set as follows: curve $A_1$ indicates an energy absorption spectrum of CFP; curve $A_2$ indicates a fluorescence emission spectrum of CFP; curve $B_1$ indicates an energy absorption spectrum of YFP; and curve $B_2$ indicates a fluorescence emission spectrum of YFP. In FIG.7, a hatched area indicates a wavelength band in which YFP absorbs energy of fluorescence emitted by CFP and FRET is generated.

Generally in FRET, the donor molecules are excited by laser and a part of the excited donor molecule emits fluorescence, while a portion of energy of the excited donor molecules is transferred towards the acceptor molecule by the coulomb interaction. The energy transfer occurs between quite close positions through a clearance of 2 nm or less. The energy transfer indicates interaction (bonding) of molecules. Therefore, when the energy transfer occurs, the acceptor molecule is excited and emits fluorescence. As shown in FIG.7, it is necessary for FRET generation that a wavelength band that the donor molecule emits fluorescence and a wavelength band that the acceptor molecule absorbs energy partially overlap.

In the present invention, as illustrated in FIG.8, the donor molecule emits fluorescence in response to irradiation of laser whose intensity is modulated at frequency f+Δf. Thereby the acceptor molecule is excited and emits fluorescence whose intensity varies at frequency f+Δf. Simultaneously, the acceptor molecule emits fluorescence whose intensity varies at frequency f in response to irradiation of laser whose intensity is modulated at frequency f. Thus, fluorescence with frequency f+Δf and fluorescence with frequency f are simultaneously received. However, fluorescence signals of fluorescences of two types can be separated based on the aforementioned different frequencies.

The FRET phenomenon can be briefly explained using the first order relaxation process as follows. Specifically, the FRET phenomenon is defined by the following formulas.

[Formula 1]

$$\frac{\partial W_d}{\partial t} = -k_{da}W_d - k_d W_d + pe^{j\omega t} \qquad \cdots (1)$$

$$\frac{\partial W_a}{\partial t} = k_{da}W_d - k_a W_a \qquad \cdots (2)$$

Settings of variables and coefficients are as follows: $W_d$ indicates density of excited donor molecules; $W_a$ indicates density of excited acceptor molecules; $k_d$ indicates fluorescence emission speed of the donor molecules; $k_a$ indicates fluorescence emission speed of the acceptor molecules; and $k_{da}$ indicates FRET speed. Additionally, $pe^{j\omega t}$ indicates density of the donor molecules to be excited by laser in a unit time whereas $\omega$ indicates $2\pi f$ where f is an intensity modulation frequency (angular frequency).

$W_d$ and $W_a$ are herein expressed by the following formulas (3), (4) using angular frequency $\omega$.

[Formula 2]

$$W_d = p \cdot \frac{(k_{da} + k_d - j\omega)}{(k_{da} + k_d)^2 + \omega^2} \cdot e^{j\omega t} \quad \cdots (3)$$

$$W_a = p \cdot \frac{k_{da} \cdot \{k_a \cdot (k_d + k_{da}) - \omega^2 - j\omega(k_a + k_d + k_{da})\}}{(\omega^2 + k_a^2) \cdot \{\omega^2 + (k_d + k_{da})^2\}} \cdot e^{j\omega t} \quad \cdots (4)$$

Phases of $W_d$ and $W_a$ in the formulas (3), (4) are expressed by the following formulas (5), (6), respectively.

On the other hand, fluorescence is expressed by the following formula (7) when laser directly excites the acceptor molecule and the acceptor molecule accordingly emits fluorescence.

[Formula 3]

$$\tan\theta_a = \frac{\omega}{k_{da} + k_d} \qquad \cdots (5)$$

$$\tan\theta_{fret} = \frac{\omega \cdot (k_a + k_d + k_{da})}{k_a \cdot (k_d + k_{da}) - \omega^2} \qquad \cdots (6)$$

[Formula 4]

$$\frac{\partial W_a}{\partial t} = -k_a W + p e^{j\omega\tau} \qquad \cdots (7)$$

When the acceptor molecule emits fluorescence when being excited in response to laser irradiation, $W_a$ is expressed by the following formula (8) derived from the formula (7) and a phase is expressed in the following formula (9).

[Formula 5]

$$W_a = p \cdot \frac{(k_a - j\omega)}{\omega^2 + k_a^2} \cdot e^{j\omega t} \qquad \cdots (8)$$

[Formula 6]

$$\tan\theta_a = \frac{\omega}{k_a} \qquad \cdots (9)$$

When a ratio of phases is computed based on the formulas (6), (9), the following formula (10) is derived.

[Formula 7]

$$\text{Ratio} = \frac{\tan\theta_{fret}}{\tan\theta_a} = \frac{k_a \cdot (k_a + k_d + k_{da})}{k_a \cdot (k_a + k_{da}) - \omega^2} \qquad \cdots (10)$$

The ratio to be calculated by the formula (10) indicates a ratio of a phase delay of fluorescence emitted from the acceptor molecules through FRET to a phase delay to be caused in direct laser irradiation to the acceptor molecules, the FRET being generated in response to laser irradiation to the donor molecules. When FRET is generated, a phase delay to be actually measured has a value roughly approximate to the formula (10). It is determined that FRET is generated if the ratio of the phase delay and a preliminarily set threshold are compared and the ratio of the phase delay is greater than the threshold.

Especially, relation "$k_d, k_a \gg k_{da}$" is established for many of the donor molecules and the acceptor molecules. Therefore, the formula (10) can be simply expressed in the following formula (11) where the ratio can be expressed with $k_d$, $k_a$. Furthermore, the ratio is expressed by the following formula (12) where $\omega^2 \ll k_d \cdot k_a$.

Especially, when the emission speeds $k_d$, $k_a$ are relatively identical to each other, the ratio will be 2 as a result of computation in the formula (12). Therefore, the analysis device 80 can precisely determine whether or not FRET is generated by computing a ratio of phase delays (i.e., a measurement result of fluorescence emitted from the acceptor molecules) and then comparing the computed ratio with a preliminarily set threshold. When FRET is not generated, the ratio of phase delays will be equal to or less than the threshold.

[Formula 8]

Where $k_{da} \ll k_d, k_a$,

$$\text{Ratio} = \frac{\left(\dfrac{k_a}{k_d}\right) + 1}{1 - \dfrac{1}{k_a \cdot k_d} \cdot \omega^2} \qquad \cdots (11)$$

[Formula 9]

Where $\omega^2 \ll k_a \cdot k_d$,

$$\text{Ratio} = \frac{k_a}{k_d} + 1 \qquad \cdots (12)$$

In the flow cytometer 10, a ratio can be computed between a phase delay of fluorescence to be emitted only by the donor molecule in response to laser irradiation and a phase delay of fluorescence emitted from the donor molecule in the course of FRET generated between the donor molecule and the acceptor molecule in response to laser irradiation. When the ratio of phase delays in the acceptor molecule is 2 as described above, however, the ratio of phase delays in the donor molecule is approximately 1. Therefore, FRET detection will be more difficult than a case that FRET is detected based on the ratio of phase delays in the acceptor molecule. In response to this, the ratio of phase delays of fluorescences emitted from the acceptor molecule is computed in the present invention. Obviously, FRET can be comprehensively detected using the ratio of phase delays of fluorescences emitted from the donor molecule in addition to the ratio of phase delays of fluorescences emitted from the acceptor molecule.

In a conventional method, laser for exciting the donor molecule is irradiated to the donor molecule and intensity of fluorescence emitted from the acceptor molecule through FRET is measured. In the method, however, the acceptor molecule may emit fluorescence when being excited by the laser for exciting the donor molecule. In other words, fluorescence emitted from the acceptor molecule when it is excited by the laser for exciting the donor molecules is excessively received in addition to fluorescence emitted from the acceptor molecule when it is excited through FRET. Therefore, a drawback comes out that FRET is falsely detected when generation efficiency of FRET is low. In the present invention, however, laser for exciting the donor molecule and the laser for exciting the acceptor molecule are used. Intensity modulation is executed for each of the lasers at a predetermined frequency. Additionally, intensity modulation frequencies are herein different by $\Delta f$ for discriminating fluorescences. Furthermore, after fluorescences to be emitted due to the laser irradiations are received, computations are executed for a phase delay of fluorescence emitted from the acceptor molecule through FRET and a phase delay of fluorescence emitted from the acceptor molecule when it is excited by laser. FRET generation is confirmed using the phase delays. As described above, the ratio of phase delays relatively largely varies depending on whether or not FRET is generated. Therefore, FRET generation can be precisely detected even if FRET efficiency is low. Furthermore, FRET can be detected by executing a signal processing in the flow cytometer. Therefore, it is possible to efficiently measure the samples in a short period of time, statistically integrate the FRET detection results, and research interactions of molecules of interest.

The method of and the device for detecting FRET of the present invention have been explained above. However, the present invention is not limited to the aforementioned embodiment and a variety of changes and modifications are obviously possible for the aforementioned embodiment without departing from the scope of the present invention.

**Claims**

1. A FRET detection method of detecting FRET (fluorescence resonance energy transfer) where energy of a first molecule transfers to a second molecule, by receiving a fluorescence emitted from the second molecule, the second molecule emitting the fluorescence when the second molecule is excited by a fluorescence emitted from the first molecule, the first molecule emitting the fluorescence when the first molecule is excited by irradiation of laser, the FRET detection method comprising the steps of:

   irradiating a first laser for exciting the first molecule and a second laser for exciting the second molecule, the first laser having an intensity modulated at a first frequency, the second laser having an intensity modulated at a second frequency, the second frequency being different from the first frequency;
   receiving the fluorescence emitted from the second molecule; and
   extracting first and second phase delays and determining whether or not an energy transfer from the first molecule to the second molecule is generated based on the first and second phase delays, in which the first phase delay represents a delay of a signal component at the first frequency, with respect to modulation of the intensity of the first laser, of a fluorescence signal obtained from the received fluorescence emitted from the second molecule, and the second phase delay represents a delay of a signal component at the second frequency, with respect to modulation of the intensity of the second laser, of the fluorescence signal obtained from the received fluorescence emitted from the second molecule.

2. The FRET detection method according to claim 1, wherein the energy transfer is determined based on a ratio of the first phase delay to the second phase delay.

3. The FRET detection method according to claim 1 or 2,
   wherein the intensity of the first laser is modulated using a first modulation signal having the first frequency for modulating the intensity of the first laser,
   the intensity of the second laser is modulated using a second modulation signal having the second frequency for modulating the intensity of the second laser,
   the first modulation signal having the first frequency is obtained by mixing the second modulation signal having the second frequency with a generation signal having a delta frequency, and
   the fluorescence signal of the received fluorescence emitted from the second molecule is sampled using a frequency of integer multiple of the delta frequency as a sampling frequency in synchronization with the generation signal having the delta frequency.

4. The FRET detection method according to claim 3, a signal component of the first frequency in the fluorescence signal and a signal component of the second frequency in the fluorescence signal are extracted by executing a frequency analysis for the sampled fluorescence signal using one integer-th of the delta frequency as a frequency resolution.

5. The FRET detection method according to any one of claims 1 to 4, wherein, when the fluorescence emitted from the second molecule is received, the fluorescence emitted by the first molecule is further received; a third phase delay of a fluorescence signal obtained from the received fluorescence emitted from the first molecule with respect to modulation of the intensity of the first laser, is computed; and the third phase delay is used for determining whether or not the FRET is generated.

6. A FRET detection device for detecting FRET where energy of a first molecule transfers to a second molecule, by receiving a fluorescence emitted from the second molecule, the second molecule emitting the fluorescence when the second molecule is excited by a fluorescence emitted from the first molecule, the first molecule emitting the fluorescence when the first molecule is excited by irradiation of laser, the FRET detection device comprising:

a laser light source unit for irradiating a first laser to the first molecule for exciting the first molecule and irradiating a second laser to the second molecule for exciting the second molecule;

a light receiving unit for receiving the fluorescence from the second molecule;

a light source control unit for generating modulation signals, for modulating an intensity of the first laser irradiated by the laser light source unit at a first frequency and for modulating an intensity of the second laser irradiated by the laser light source at a second frequency, the second frequency being different from the first frequency; and

a processing unit for extracting a first phase delay and a second phase delay and determining whether or not an energy transfer from the first molecule to the second molecule is generated based on the first and second phase delays, in which the first phase delay represents a delay of a signal component at the first frequency, with respect to modulation of the intensity of the first laser, of a fluorescence signal obtained from the received fluorescence emitted from the second molecule, and the second phase delay represents a delay of a signal component at the second frequency, with respect to modulation of the intensity of the second laser, of a fluorescence signal obtained from the received fluorescence emitted from the second molecule.

7. The FRET detection device according to claim 6, wherein the processing unit determines whether or not the energy transfer is generated based on a ratio of the first phase delay to the second phase delay.

8. The FRET detection device according to claim 6 or 7,

wherein the light source control unit modulates the intensity of the first laser using a first modulation signal having a first frequency for modulating the intensity of the first laser,

the light source control unit modulates the intensity of the second laser using a second modulation signal having a second frequency for modulating the intensity of the second laser,

the first modulation signal having the first frequency is obtained by mixing the second modulation signal having the second frequency with a generation signal having a delta frequency, and

the processing unit samples the fluorescence signal of the received fluorescence emitted from the second molecule using integer multiple of the delta frequency as a sampling frequency in synchronization with the generation signal having the delta frequency.

9. The FRET detection device according to claim 8, wherein the processing unit extracts a signal component of the fluorescence signal having the first frequency and a signal component of the fluorescence signal having the second frequency by executing a frequency analysis for the sampled fluorescence signal using one integer-th of the delta frequency as a frequency resolution.

10. The FRET detection device according to any one of claims 6 to 9,

wherein the light receiving unit receives the fluorescence emitted from the first molecule in addition to the fluorescence emitted from the second molecule, and

the processing unit computes a third phase delay and uses the third phase delay for determining whether or not FRET is generated, the third phase delay of a fluorescence signal obtained from the fluorescence, which is emitted from the first molecule and received by the light receiving unit, with respect to modulation of the intensity of the first laser.

SEATH        SAMPLE     ⬡ ⌒12

30

26

22        20

24

28

CONTROL AND
PROCESSING
SECTION

32

ANALYSIS
DEVICE
(COMPUTER)

80

10

# FIG.1

22a

DONOR EXCITATION
LIGHT SOURCE

23a

30   12

ACCEPTOR EXCITATION
LIGHT SOURCE

22b

23b

LASER
DRIVER

LASER
DRIVER

34a

34b

40

40

22

LASER LIGHT SOURCE SECTION

## FIG.2

26

30   12

26b

27b

26a

26c₁

26c₂

27a

28

28

## FIG.3

CONTROL AND PROCESSING SECTION

SIGNAL
GENERATION
UNIT
40

28

50        48        46

34a:LASER DRIVER ← $f + \Delta f$ — SSB — POWER SPLITTER — $f$ — OSCILLATOR

34b:LASER DRIVER ← $f$

42
SIGNAL
PROCESSING
UNIT

27a → AMP 54a → IQ MIXER 58a

POWER SPLITTER 56

$\Delta f$ OSCILLATOR 47

27b → AMP 54b → IQ MIXER 58b

AMP ~52

→ 34a, 34b : LASER DRIVER

TRIGGER SIGNAL ___ 24 : LIGHT RECEIVING UNIT

LOW PASS FILTER ~62

SYSTEM CONTROLLER ~60

$n \cdot \Delta f$

AMP      A/D → 80 : ANALYSIS DEVICE (COMPUTER)

44
CONTROLLER        64        66

FIG.4

17

FIG.5

START

LASER IRRADIATION
TO SAMPLE — STEP S10

RECEPTION OF FLUORESCENCE FROM
DONOR MOLECULE AND ACCEPTOR
MOLECULE, AND PROCESSING
OF FLUORESCENCE SIGNAL — STEP S20

SAMPLING OF PROCESSING
SIGNAL IN FREQUENCY n·Δf — STEP S30

FREQUENCY ANALYSIS — STEP S40

DETECTION OF FRET — STEP S50

ESTIMATION FOR
PREDETERMINED NUMBER
OF SAMPLES? — STEP S60

NO

YES

END

# FIG.6

FLUORESCENCE
INTENSITY

$A_1$   $A_2$   $B_1$ $B_2$

325 350 375 400 425 450 475 500 525 550 575 600 625 650 675 700
WAVELENGTH (nm)

# FIG.7

$f + \Delta f$                    $f$

$f + \Delta f$

DONOR
MOLECULE   $f + \Delta f$   ACCEPTOR
MOLECULE

$f$

# FIG.8

## INTERNATIONAL SEARCH REPORT

| | International application No. |
| --- | --- |
| | PCT/JP2007/066850 |

A. CLASSIFICATION OF SUBJECT MATTER
*G01N21/64*(2006.01)i

According to International Patent Classification (IPC) or to both national classification and IPC

B. FIELDS SEARCHED

Minimum documentation searched (classification system followed by classification symbols)
G01N21/62-G01N21/83, G01N33/48-G01N33/98

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
| --- | --- | --- |
| A | JP 10-227739 A (Kabushiki Kaisha Laboratory of Molecular Biophotonics), 25 August, 1998 (25.08.98), Full text; Figs. 1 to 6 (Family: none) | 1-10 |
| A | JP 10-332594 A (Kabushiki Kaisha Laboratory of Molecular Biophotonics), 18 December, 1998 (18.12.98), Full text; Figs. 1 to 7 (Family: none) | 1-10 |

| ☒ Further documents are listed in the continuation of Box C. | ☐ See patent family annex. |
| --- | --- |

| * Special categories of cited documents: | | "T" later document published after the international filing date or priority date and not in conflict with the application but cited to understand the principle or theory underlying the invention |
| --- | --- | --- |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| 09 November, 2007 (09.11.07) | 20 November, 2007 (20.11.07) |

| Name and mailing address of the ISA/ | Authorized officer |
| --- | --- |
| Japanese Patent Office | |
| Facsimile No. | Telephone No. |

Form PCT/ISA/210 (second sheet) (April 2007)

**EP 2 196 795 A1**

<table>
<tr><td colspan="2" align="center"><strong>INTERNATIONAL SEARCH REPORT</strong></td><td>International application No.<br>PCT/JP2007/066850</td></tr>
<tr><td colspan="3">C (Continuation).    DOCUMENTS CONSIDERED TO BE RELEVANT</td></tr>
<tr><td>Category*</td><td>Citation of document, with indication, where appropriate, of the relevant passages</td><td>Relevant to claim No.</td></tr>
<tr><td align="center">A</td><td>JP 7-229835 A  (Hamamatsu Photonics Kabushiki<br>Kaisha),<br>29 August, 1995 (29.08.95),<br>Full text; Figs. 1 to 15<br>& US 5776782 A          & EP 668498 A3<br>& DE 69531515 D</td><td align="center">1-10</td></tr>
</table>

Form PCT/ISA/210 (continuation of second sheet) (April 2007)

22

**EP 2 196 795 A1**

## REFERENCES CITED IN THE DESCRIPTION

**Patent documents cited in the description**

- JP 2005207823 A